# EUROPEAN PATENT SPECIFICATION

(11) **EP 2 105 510 B1**
(45) Date of publication and mention of the grant of the patent: **02.02.2011**
(21) Application number: 06841777.3
(22) Date of filing: 29.12.2006
(51) Int. Cl.: C12Q 1/68

(54) **METHOD FOR DETECTION OF MANDARIN**
VERFAHREN ZUM NACHWEIS VON MANDARINEN
PROCÉDÉ DE DÉTECTION DE MANDARINE

(43) Date of publication of application: 30.09.2009
(73) Proprietor: Fundacion Azti-azti Fundazioa, 48395 Vizcaya (ES)
(72) Inventor: LÓPEZ DE GAMBOA, Itziar, E-48395 Sukarrieta (Vizcaya) (ES); PARDO GONZÁLEZ, Miguel Ángel, E-48395 Sukarrieta (Vizcaya) (ES)
(74) Representative: Ezcurra Zufia, Maria Antonia
(86) International application number: PCT/ES2006/000725
(87) International publication number: WO 2008/081052

(56) References cited:
- WO-A-98/14607
- DE-A1- 10 239 585
- FR-A1- 2 876 378
- LIU YU-SHAN ET AL: "Adulteration identification of citrus juice by denaturing gradient gel electrophoresis (DGGE)" JOURNAL OF FOOD AND DRUG ANALYSIS, vol. 14, no. 1, March 2006 (2006-03), pages 44-49, XP008083454 ISSN: 1021-9498
- DE ARAUJO E F ET AL: "What is Citrus? Taxonomic implications from a study of cp-DNA evolution in the tribe Citreae (Rutaceae subfamily Aurantioideae)" ORGANISMS DIVERSITY & EVOLUTION, ELSEVIER, vol. 3, no. 1, 2003, pages 55-62, XP004960888 ISSN: 1439-6092
- MOONEY RACHEL ET AL: "Evaluation of a polymerase chain reaction-based heteroduplex assay for detecting the adulteration of processed orange juice with mandarin juice." JOURNAL OF AOAC INTERNATIONAL 2006 JUL-AUG, vol. 89, no. 4, July 2006 (2006-07), pages 1052-1060, XP008083087 ISSN: 1060-3271

## Description

### OBJECT OF THE INVENTION

The present invention refers to a method for detecting mandarin preferably in commercial orange juices, either consisting of direct juice or made from concentrates.

The object of the method is to ascertain whether the orange juice has been adulterated with mandarin juice.

For the invention method specific DNA probes have been developed.

### BACKGROUND TO THE INVENTION

Different methods of detecting citruses are known:
Methods based on chemical markers for detecting adulteration of orange juice (*Citrus sinensis*) with mandarins (*Citrus reticulata, C. unshiu, C. deliciosa, C. clementina* and also hybrids).

These methods based on chemical markers have been developed due to the fact that in recent years the profiles of minerals, organic acids, sugars, flavonoids, carotenoids, free amino acids etc. have been studied and different profiles in a group of polymethoxyflavones have been found between *C. sinensis* and *C*. *reticulata.* However these results are not conclusive.

One of the analytical methods used to detect the presence of adulterants in fruit juices is the method of analysing the content of soluble solids (Brix degrees) by means of indirect refractometry testing which establishes a minimum level of Brix degrees for each juice. (Methods of analysis and sampling CODEX STAN 247/2005).

Nevertheless, in the case of the juices obtained from orange the Brix degrees level overlaps with that obtained from mandarins. Therefore, we are in a position to conclude that to date none of these methods has been able to resolve the problem of adulteration of orange juice with mandarin juice.

As we have just indicated, the use of chemical markers has been unable to resolve the problem of adulteration of orange juices with other citruses such as mandarin.

In recent years some attempts have been made to resolve the problem by using biological markers such as DNA and proteins. The first projects were based on differences between the composition and structure of certain proteins of the exocarp, mesocarp and endocarp of citruses which generate different immunogenicity between oranges and lemons.

An immunoassay was subsequently developed to determine the content of juice in commercial samples using serum as opposed to the total content of orange protein. However, these methods were not sufficiently specific to distinguish between citruses.

A step forward was the development of an effective serum for detecting a specific peptide of orange skin. However, the serum was not sufficiently specific to oranges since cross reactions with mandarin peptides were detected. These projects based on protein differences have not provided good results. This is partly due to the fact that the proteins cannot be constant in all the fruit tissues, as they may be affected by the maturation process and/or growing conditions. These circumstances make it very easy to encounter cross reactions with proteins present in other species due to the low specificity of these detection systems. Another weak point in the use of proteins as markers is the fact that when we work with samples of commercial juices these have been subjected to heat pasteurisation in the packaging process which degrades the proteins, thus modifying their properties.

In recent years several molecular techniques have been developed, based on an analysis of DNA, for the purpose of identifying the species of origin in numerous sectors of the agricultural food trade. The DNA molecule is much more stable (for example in pasteurisation) and also contains more information available for analysis. For this and other reasons molecular techniques based on DNA analysis are currently attracting the attention of the scientific community in respect of identifying ingredients. In respect of the application of DNA analysis techniques in citruses, this has been focused on the development of molecular markers to map characters and genes of interest, or to resolve phylogenetic implications in plants.

With respect to the use of genetic markers in species belonging to the Citrus genus, phylogenetic implications are inferred following analysis of chloroplast spaced fragments((trnL-trnF and trnT-trnL).

In addition, various chloroplast markers have been described which are extremely useful for discriminating between phylogenetically similar species of citruses.

The patent with publication number GB2283568 describes the application of molecular markers to detect adulterations and develops a method based on an RAPD system (Random Amplified Polymorphic DNA analysis) which differentiates some species of citruses; however, it is unable to discriminate between phylogenetically similar species and certainly not between hybrids.

Finally, in an article by Mooney, R., Chappel, L. Knight, A. I., "Evaluation of a polymerase chain reaction-based heteroduplex assay for detecting the adulteration of processed orange juice with mandarin juice". 2006 Journal of AOAC International. 89(4): 1052-1060 based on the patent WO98/14607, a method has been developed for detecting the presence of varieties of mandarin *C*. *reticulata* in orange juices by means of PCR-heteroduplex technology. However, this work excludes analysis of Clementine mandarin varieties (*C. clem* entina) and satsumas (*C. unshiu*) which are extremely important to the sector.

Furthermore Liu Yu-Shan et al. in JOURNAL OF FOOD AND DRUG ANALYSIS, vol. 14, no.1, March 2006 pages 44-49 discloses a method for detecting mandarin in orange juices comprising the PCR amplification of a fragment of the trnL gene.

### DESCRIPTION OF THE INVENTION

The authenticity of juices and nectar is extremely important due to the fact that it is very easy to adulterate a juice by adding a foreign substance. Adulteration of orange juice with juices and/or pulp from other fruits, mainly citruses such as mandarin, is gaining in significance as a result of fluctuations in the orange production in terms of quality and quantity.

The problem resolved in the present invention is through development of a method for determining the adulteration of orange juices with mandarin which can be reproduced and standardised.

Currently known methods, which include electrophoretic analysis, are difficult to standardise.

The method in this invention uses Real Time PCR technology, and is rapid, easy to standardise and potentially automatable, which makes it ideal as a reference technique in quality control laboratories.

The invention method has included varieties of Clementine mandarin (*C. clementina*) and satsumas (*C. unshiu*) which are extremely important in the sector. The varieties of orange and mandarin analysed are as follows: *Citrus clementina* Hort. (varieties: MARISOL, ORONULES, OROVAL, ESBAL, CLEMENLATE, CLEMENSO, FINA, HERNANDINA, LORETINA, CLEMENPONS, ARRUFATINA, ORO GRANDE y CLEMENULES) *Citrus sinensis* (L.) Osb (varieties: LANE LATE, NAVELATE, NAVELINA, VALENCIA LATE, BURJASOT, SOLITA y SALUSTIANA GIL) *Citrus deliciosa, Citrus unshiu* K. (varieties: OKITSU, SATSUMA 4n and CLAUSELLINA) hybrids (varieties FORTUNE and ORTANIQUE).

The invention is based on the amplification of an 83 pb fragment of the chloroplast trnL transfer gene using a pair of specific primers and their simultaneous detection in a Real Time PCR system with two specific probes.

The selected primers R22-L, SEQ ID N°1 (5'-AACTCGATAAAGGATGAAGGATAAGG-3') and R22-H, SEQ ID N°2 (5'-CGTATTTGGTTGTGATTTTTGAGTTAG-3'), and the probes TaqMan MGB R22vic SEQ ID N°3 (5'-CGC TGC GTA TAC ATA GTC TAT A-3') specific to oranges and R22fam SEQ ID N°4 (5'-CGC TGC GTA TCC ATA GTC TAT A -3) specific to mandarins were designed having amplified, sequenced, and aligned the R2 space region (with 650 pb) of different citruses following the specifications previously described by Tarbelet P.; Gielly L.; Pautou G. and Bouvet J. Universal primers for amplification of three non-coding regions of chloroplast DNA. Plant Molecular Biology. 1991. 17: 1105-1109.

The R2 fragment sequences of the varieties of orange and mandarin which have been amplified, sequenced and aligned are shown below
R2_C.clementine_OROVAL, SEQ ID n° 5
R2_C.clementine_HERNANDINA, SEQ ID n° 6
R2_C.unshiu_CLAUSELLINA, SEQ ID n° 7
R2_C.sinensis_VALENCIA LATE, SEQ ID n° 8
R2_C.clementine_FINA, SEQ ID n°9
R2_C.clementine_ESBAL, SEQ ID n° 10
R2_C.clementine_ARRUFATINA, SEQ ID n° 11
R2_C.sinensis_NAVELATE(1), SEQ ID n° 12
R2_Hybrids_FORTUNE, SEQ ID n° 13
R2_C.clementine_MARISOL, SEQ ID n° 14
R2_C.sinensis_SALUSTIANA(1), SEQ ID n° 15
R2_C.clementine_ORONULES, SEQ ID n° 16
R2_C.deliciosa_COMMON, SEQ ID n° 17
R2_C.unshiu_OKITSU, SEQ ID n° 18
R2_C.sinensis_LANE LATE, SEQ ID n° 19
R2_Hybrids_ORTANIQUE, SEQ ID n° 20
R2_C.sinensis_NAVELINA(1), SEQ ID n° 21
R2_C.clementine_LORETINA, SEQ ID n° 22
R2_C.clementine_CLEMENPONS, SEQ ID n° 23
R2_C.clementine_CLEMENSOL, SEQ ID n° 24
R2_C.unshiu_SATSUMA(1), SEQ ID n° 25
R2_C.clementine_CLEMENLATE, SEQ ID n° 26
R2_C.clementine_CLEMENULES, SEQ ID n° 27
R2_C.clementine_ORO GRANDE, SEQ ID n° 28
R2_C.unshiu_SATSUMA(2), SEQ ID n° 29
R2_C.sinensis_NAVELINA(2), SEQ ID n° 30
R2_C.sinensis_NAVELATE(2), SEQ ID n° 31
R2_C.sinensis_SALUSTIANA(2), SEQ ID n° 32 R2_C.sinensis_SOLITA, SEQ ID n° 33

The alignments of the varieties of orange and mandarin are shown below marking the characterised polymorphism with a box.
R22-L SEQ ID N°1 AACTCGATAAAGGATGAAGGATAAGG
R22-H SEQ ID N°2 CTAACTCAAAAATCACAACCAAATACG

### Mandarins

R2_C.clementine_FINA, SEQ ID n° 34
R2_C.clementine_CLEMENSOL,SEQ ID n° 35
R2_{_}C.unshiu-CLAUSELLINA, SEQ ID n° 36
R2_C.clementine_OROVAL, SEQ ID n° 37
R2_C.clementine_ESBAL, SEQ ID n° 38
R2_C.clementine_CLEMENLATE, SEQ ID n° 39
R2_C.clementine_HERNANDINA, SEQ ID n° 40
R2_C.clementine_ARRUFATINA, SEQ ID n° 41
R2_C.clementine_MARISOL, SEQ ID n° 42
R2_C.clementine_ORO, SEQ ID n° 43
R2_C.unshiu_OKITSU, SEQ ID n° 44
R2_Hybrids_FORTUNE, SEQ ID n° 45
R2_C.unshiu_SATSUMA_2_, SEQ ID n° 46
R2_C.clementine_ORONULES, SEQ ID n° 47
R2_C.clementine_LORETINA, SEQ ID n° 48
R2_Hybrids_ORTANIQUE, SEQ ID n° 49
R2_C.clementine_CLEMENPONS, SEQ ID n° 50
R2_C.clementine_CLEMENULES, SEQ ID n° 51
R2_C.unshiu_SATSUMA_1_, SEQ ID n° 52
R2_C.deliciosa_COMMON, SEQ ID n° 53
Oranges
   R2_C.sinensis_SALUSTIANA_2_, SEQ ID n° 54
R2_C.sinensis_LANE, SEQ ID n° 55
R2_C.sinensis_NAVELINA_2_, SEQ ID n° 56
R2_C.sinensis_NAVELATE_2_, SEQ ID n° 57
R2_C.sinensis_SOLITA, SEQ ID n° 58
R2_C.sinensis_SALUSTIANA_1_, SEQ ID n° 59
R2_C.sinensis_NAVELINA_1_, SEQ ID n° 60
R2_C.sinensis_NAVELATE_1_, SEQ ID n° 61
R2_C.sinensis_VALENCIA, SEQ ID n° 62

The invention method presents a detection limit of 5% without having detected any false positive or negative, following analysis of over 50 samples of orange juice adulterated with between 5-20% de mandarin.

In addition, as the invention method uses specific probes it increases the specificity of the system, thus eliminating possible false positives.

As the size of the amplified diagnostic fragment is only 83 pb, this method may be effectively used with direct or pasteurised juices where the DNA has degraded.

In addition the speed of the diagnostic fragment detection method using a fluorescent probe, slightly reduces analysis times.

The use of this detection system also eliminates cross contaminations between different samples as detection is made in the same sealed tube where the fragment is amplified without any further handling.

The present invention method has three distinct phases:
- Extraction and purification of the DNA
- Checking of the quantity and quality of the DNA
- Determination of the presence of mandarin DNA in the problem sample by detecting a fragment of the chloroplast trnL transfer gene.

In order to avoid contamination with other DNA not from the sample and the presence of DNAsas in laboratory material all the reactants and materials to be used are sterilised. The sample is homogenised and treated with a solution of alkaline lysis in the presence of proteinases. The DNA is subsequently purified from the lysates using resins which retain the DNA.

The extracted DNA should be in sufficient quantity and quality for analysis. The concentration of DNA in the samples is calculated spectrophotometrically at E260 nm. With respect to the DNA quality, although highly processed samples are treated in which the DNA is very degraded, this is not a problem as the size of the fragment amplified in the Real Time PCR system is sufficient in length (83 pb) to be found in abundance even in the most degraded samples.

Detection of the specific fragment is made in a sealed tube in the presence of primers R22-L SEQ ID N°1 and R22-H SEQ ID N°2 of the TaqMan MGB probes namely R22vic SEQ ID N°3 and R22fam SEQ ID N°4 and the necessary reactants to carry out the amplification. While the amplification takes place the probes specifically hybridise to the strands of the recently synthesised fragment. Immediately after a DNA polymerase enzyme (present in the reaction medium) begins to duplicate the strand degrading in its path the probes, emitting FAM oR VIC type fluorescence which is finally differentially detected by an optical detection system incorporated in the Real Time PCR. The fluorescence emitted by the R22vic SEQ ID N°3 probe is specific to orange DNA whereas that emitted by the R22fam SEQ ID N°4 probe is that of mandarin. The fluorescence results obtained are converted to a Ct value which indicates which cycle each fluorescence will begin to emit. The relative abundance of Ct values indicates the presence to a greater or lesser degree of specific mandarin DNA in the orange juice sample.

### PREFERRED EMBODIMENT OF THE INVENTION

In a practical example of the invention the possibility of detecting the presence of mandarin in a direct orange juice will be shown.

### 1- Extraction and purification of DNA

2 mL of direct orange juice are taken in duplicate and centrifuged to 13,000 rpm for 5 min. Following the centrifugation stage the supernatant is disposed of and 300 ml extraction buffer[SDS 1% (p/v), 150 mM NaCl, 2 mM EDTA, Tris-HCl pH 8.0], 50 µl of a solution of thiocyanate of guanidium 5 M and 30 µl of proteinase k is added to the precipitate obtained. Each of the tubes is incubated at 56°C for 1 hour. Following the incubation period, the non-dissolved material was eliminated by centrifugation (Refrigerated centrifuge 5417 C/R, Eppendorf)for 1-2 minutes at ambient temperature. Centrifugation is repeated as often as necessary until a supernatant free from organic remains in suspension is finally obtained. The resulting supernatant is treated with a commercial DNA extraction kit (Wizard-DNA CleanUp, Promega) following the manufacturer's specifications. The DNA is finally diluted in 50 µl of sterile distilled milliQ-quality water.

### 2- Verification of the quantity and quality of DNA.

In order to verify the quantity of DNA an aliquot of the DNa was taken and its absorbance was measured spectrophotometrically at 260 nm. Based on the value obtained, the concentration was estimated in ng/µl.

### 3- Determination of the presence of mandarin DNA in the problem sample by detection of a fragment of the chloroplast trnL gene.

Detection of the specific fragment of mandarin is carried out in a reaction volume of 25 µl which contains 0.75 µl of a 300 nM solution of the primer R22-L, 0.75 µl of a 300 nM solution of the primer R22-H, 2.5 µl of a 2.50 µM solution of the R22fam probe, 2.5 µl of a 2.50 µM solution of the R22vic probe, 12.5 µl of the TaqMan^{™} Universal Master Mix solution(Applied Biosystems), 40 ng of extracted DNA of problem sample and finally sterile milliQ-distilled water to adjust the final volume to 25 µl. Each replication of the sample is carried out in triplicate. In addition, a negative control is introduced into the analysis which is also duplicated (non template control, ntc.) which consists of substituting the DNA for water and two positive controls, one with 40 ng of 100 % orange DNA and another with 40 ng of 100 % mandarin DNA.

The Real Time PCR amplification programme is as follows: an initial cycle at 50°C for 2 min followed by 10 min at 95°C, in order to subsequently make 40 cycles at 95°C for 15 sec and 60°C for 1 min.

At the end of the reaction two Ct values are obtained mediated by each problem sample, one per probe. If the value for the Ct corresponding to the Fam fluorescence (emitted by the specific mandarin probe) is less that that obtained with the positive control of 100% mandarin, it may be concluded that the sample is adulterated by mandarin.

## Claims

1. Method for detecting mandarin in orange juices **characterised by** the amplification of an 83 pb fragment of chloroplast trnL transfer gene by means of a pair of specific primers mR22-L SEQ ID N°1 and R22-H SEQ ID N°2 and their simultaneous detection in a Real Time PCR system with two specific probes termed R22vic SEQ ID N°3 and R22fam SEQ ID N°4.

2. Method according to claim 1 **characterised in that** the specific probes are TaqMan MGB probes.

3. Method according to claim 1 **characterised in that** the invention comprises the three following stages:
• Extraction and purification of the DNA
• Verification of the quantity and quality of the DNA
• Determination of the presence of mandarin DNA in the problem sample.

## Patentansprüche

1. Methode fiir die Erkennung von Mandarin in Orangensaft durch die Verstärkung eines Fragments von 83 pb vom trnL Transfer Chloroplast Gen mittels eines Paars spezifischer Primers - mR22-L SEQ ID Nr.1 und R22-H SEQ ID Nr.2 - und ihrer gleichzeitigen Erkennung mit einem PCR System Echter Zeit mit zwei spezifischen Prüfern, Namen R22vic SEQ ID Nr.3 und R22fam SEQ ID Nr.4.

2. Methode entsprechend Anspruch Nr.1- **gekennzeichnet durch** die spezifischen Prüfer TaqMan MGB PROBES.

3. Methode entsprechend Anspruch Nr.1- **gekennzeichnet durch** die drei verschiedenen Phasen der Entdeckung -, namentlich:
• DNA-Entnahme -und Reinigung.
• DNA-Mengen -und Qualitätsüberprüfung.
• Feststellung der Anwesenheit von Mandarin bei der DNA-Probe.

## Revendications

1. Procédé pour la détection de mandarin en jus d'orange **caracterisée par** l'amplification d'un fragment de 83 pb de transfert de gènes chloroplastiques tmL grâce à une paire de d'amorces spécifiques mR22-L SEQ ID N°1 et R22-H SEQ ID N°2 et leurs détection simultanée en utilisant un méchanism de sondes PCR au Temps Réel avec deux sondes denominées R22vic SEQ ID N°3 et R22fam SEQ ID N°4.

2. Procédé selon la revendication 1, **caracterisée en ce que** les sondes spécifiques sont des sondes TaqMan MGB.

3. Procédé selon la revendication 1 **caracterisé en ce que** l'invention comprend les trois étapes suivantes:
• Extraction et purification de l'ADN
• Vérification de la quantité et la qualité de l'ADN
• Détermination de la présence d'ADN dans l'échantillon de mandarin.


### LISTA DE SECUENCIAS

<110> FUNDACIÓN AZTI - AZTI FUNDAZIOA
<120> MÉTODO DE DETECCIÓN DE MANDARINA
<130> 1
<160> 4
<170> PatentIn version 3.1
<210> 1
<211> 26
<212> DNA
<213> Citrus unshiu
<400> 1
   aactcgataa aggatgaagg ataagg
   26
<210> 2
<211> 27
<212> DNA
<213> Citrus unshiu
<400> 2
   cgtatttggt tgtgattttt gagttag
   27
<210> 3
<211> 22
<212> DNA
<213> Citrus unshiu
<400> 3
   cgctgcgtat acatagtcta ta
   22
<210> 4
<211> 22
<212> DNA
<213> Citrus unshiu
<400> 4
   cgctgcgtat ccatagtcta ta
   22